(19) Europäisches Patentamt
European Patent Office
Office européen des brevets

(11) EP 0 811 372 B1

(12) **FASCICULE DE BREVET EUROPEEN**

(45) Date de publication et mention de la délivrance du brevet:
**11.08.1999 Bulletin 1999/32**

(51) Int. Cl.[6]: **A61K 7/48**, A61K 7/02

(21) Numéro de dépôt: **97401184.3**

(22) Date de dépôt: **29.05.1997**

(54) **Composition de maquillage ou de soin sans transfert à alkylpolysiloxane**

Abriebfeste Schmink- oder Körperpflegezusammensetzung mit Alkylpolysiloxan

Make-up or personal care composition which does not rub off and which comprises alkylpolysiloxane

(84) Etats contractants désignés:
**DE ES FR GB IT**

(30) Priorité: **07.06.1996 FR 9607107**

(43) Date de publication de la demande:
**10.12.1997 Bulletin 1997/50**

(60) Demande divisionnaire:
**99400181.6 / 0 925 784**

(73) Titulaire: **L'OREAL**
**75008 Paris (FR)**

(72) Inventeur: **Bara, Isabelle**
**75013 Paris (FR)**

(74) Mandataire: **Lhoste, Catherine**
**L'OREAL-DPI**
**6 rue Bertrand Sincholle**
**92585 Clichy Cédex (FR)**

(56) Documents cités:
**EP-A- 0 521 647**
**EP-A- 0 530 085**
**EP-A- 0 602 905**
**WO-A-96/40044**
**FR-A- 2 688 134**
**US-A- 4 423 041**
**US-A- 5 288 482**



Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Xerox (UK) Business Services
2.16.7/3.6

## Description

La présente invention a trait à une composition notamment pour le soin et/ou le maquillage de la peau et/ou des lèvres, et en particulier un rouge à lèvres sous forme de stick ou un fond de teint sous forme de stick ou d'émulsion.

Les compositions de rouge à lèvres et de fond de teint comprennent généralement des corps gras tels que des huiles, des composés pâteux et des cires, ainsi qu'une phase particulaire généralement composée de charges et de pigments.

Ces compositions, lorsqu'elles sont appliquées sur la peau ou les lèvres, présentent l'inconvénient de transférer, c'est-à-dire de se déposer au moins en partie, en laissant une trace, sur certains supports avec lesquels elles peuvent être mises en contact, et notamment un verre, une tasse, un vêtement ou la peau. Il s'ensuit une persistance médiocre du film sur la peau ou sur les lèvres nécessitant de renouveler régulièrement l'application de la composition de fond de teint ou de rouge à lèvres. Par ailleurs, l'apparition de traces inacceptables sur certains vêtements et notamment sur les cols de chemisier peut écarter certaines femmes à utiliser ce type de maquillage.

Lié à cet inconvénient de transfert des compositions de rouge à lèvres de l'art antérieur, il faut encore noter la fâcheuse tendance des films de ces compositions à être dissous par certains plats cuisinés et notamment par les plats contenant des huiles végétales (salades à la vinaigrette en particulier).

Un autre inconvénient des compositions de rouge à lèvres de l'art antérieur réside dans la migration de ces compositions, c'est-à-dire dans la tendance de ces compositions à se propager à l'intérieur des rides et ridules de la peau qui entourent les lèvres, entraînant un effet inesthétique.

Depuis plusieurs années, de nombreux cosméticiens se sont intéressés aux compositions de rouge à lèvres et plus récemment aux compositions de fond de teint « sans transfert ». Ainsi, la société Shiseido a envisagé dans sa demande de brevet JP-A-61-65809 des compositions de rouge à lèvres « sans transfert » contenant de 1 à 70% en poids de résine liquide de silicone à motifs répétitifs silicates (ou à réseau tridimensionnel) comportant des chaînes pendantes alkylées de 1 à 6 atomes de carbone ou phénylées, de 10 à 98% en poids d'une huile de silicone volatile à chaîne SI-O cyclique et à radicaux méthyle et des charges pulvérulentes. Ces compositions, bien que tout-à-fait satisfaisantes pour la propriété de « sans transfert » présentent l'inconvénients d'être liquides et donc peu commodes à utiliser, ou tout au moins loin du concept classique d'un rouge à lèvres en bâton, limitant le nombre de femmes susceptibles d'utiliser ce type de rouge à lèvres. De plus, le film obtenu sur les lèvres après évaporation de l'huile de silicone présente l'inconvénient de devenir inconfortable au cours du temps (sensation de dessèchement et de tiraillement), écartant encore un certain nombre de femmes de ce type de rouge à lèvres. Pour améliorer le confort de ce type de composition, on pourrait ajouter des huiles non volatiles siliconées ou non, mais dans ce cas on perd en efficacité « sans transfert ».

Plus récemment, la société Revlon a envisagé dans sa demande de brevet EP-A-602905 des rouges à lèvres « non transfert » contenant une silicone volatile cyclique ou linéaire et à chaînes méthyles pendantes et une résine de silicone comportant une chaîne estérifiée pendante ayant au moins 12 atomes de carbone. Le film de rouge à lèvres a encore l'inconvénient de manquer de confort à l'application et notamment d'être trop sec.

La présente invention a justement pour objet une composition de soin ou de maquillage permettant de remédier à ces inconvénients et permettant en particulier l'obtention d'un film ne transférant pas, ne migrant pas et présentant des propriétés cosmétiques améliorées par rapport à celles des produits « sans transfert » de l'art antérieur notamment des propriétés de glissant, de non tiraillement et de non dessèchement des lèvres.

Il est par ailleurs connu du document US-A-5 288 482, des compositions de rouges à lèvres contenant des polymères siliconés à chaîne alkyle, du document FR-A- 2 688 134 des poudres cosmétiques contenant comme liant une huile de silicone, une cire de silicone et une résine de silicone et, du document EP-A-0 530 085 des rouges à lèvres contenant une dispersion de cire de silicone dans une phase aqueuse.

L'invention s'applique non seulement aux produits de maquillage des lèvres mais aussi aux produits de soin des lèvres ainsi qu'aux produits de maquillage et de soin de la peau tels que les fonds de teint. En effet, les produits de maquillage du visage présentent les mêmes inconvénients de « transfert » sur un support que les rouges à lèvres.

Ainsi, l'invention a pour objet une composition de maquillage ou de soin sans transfert telle que définie dans les revendications.

La composition de l'invention à l'avantage de pouvoir se présenter sous forme solide et par exemple sous forme d'un stick. En outre, elle permet l'obtention d'un film homogène, facilement applicable, s'étalant aisément et uniformément. Le film obtenu présente également une texture légère et reste confortable, non sec, et peut être porter tout au long de la journée. Avantageusement, la composition de l'invention se présente sous forme anhydre.

L'utilisation d'une cire de silicone à chaîne alkyle et à caractère polymérique apporte une certaine élasticité au film le rendant plus confortable. En outre, l'association d'une silicone volatile et d'une cire de silicone, toutes deux à chaîne alkyle assure une bonne compatibilité et une bonne homogénéité du mélange lors de la fabrication de la composition, sans exsudation d'huile (en cas de présence d'huile) ou cristallisation de cire au cours de la fabrication et du temps. En outre, cette association de silicones alkylées permet d'introduire dans la composition aussi bien des adjuvants hydrocarbonés que des adjuvants siliconés à chaîne alkyles linéaires ou ramifiées comme des copolymères et donc d'adapter les propriétés du film notamment en ce qui concerne le confort sur les lèvres ou la peau des êtres humains. Les adjuvants doivent bien entendu ne pas nuire à l'homogénéité, la stabilité et à la propriété ⟨⟨non transfert⟩⟩ de la composition.

De façon préférentielle, la composition de l'invention peut comprendre une phase particulaire, généralement présente à raison de 0 à 35 % du poids total de la composition, de préférence de 5 à 25 %, et qui peut comprendre des pigments et/ou des nacres et/ou des charges habituellement utilisés dans les compositions cosmétiques.

Par pigments, il faut comprendre des particules blanches ou colorées, minérales ou organiques, insolubles dans la cire et la silicone volatile, destinées à colorer et/ou opacifier la composition. Par charges, il faut comprendre des particules incolores ou blanches, minérales ou de synthèse, lamellaires ou non lamellaires. Par nacres, il faut comprendre des particules irisées, notamment produites par certains mollusques dans leur coquille ou bien synthétisées. Ces charges et nacres servent à modifier la texture de la composition ainsi que l'effet de matité/brillance.

Les pigments peuvent être présents dans la composition à raison de 0 à 25 % du poids de la composition finale, et de préférence à raison de 5 à 15 %. Comme pigments minéraux utilisables dans l'invention, on peut citer les oxydes de titane, de zirconium ou de cérium ainsi que les oxydes de zinc, de fer ou de chrome et le bleu ferrique. Parmi les pigments organiques utilisables dans l'invention, on peut citer le noir de carbone, et les laques de baryum, strontium, calcium, aluminium.

Les nacres peuvent être présentes dans la composition à raison de 0 à 20 % du poids total de la composition, de préférence à un taux élevé de l'ordre de 8 à 15 %. Parmi les nacres utilisables dans l'invention, on peut citer le mica recouvert d'oxyde de titane, d'oxyde de fer, de pigment naturel ou d'oxychlorure de bismuth tel que le mica titane coloré.

Les charges peuvent être présentes à raison de 0 à 35 % du poids total de la composition, de préférence 5 à 15 %. On peut notamment citer le talc, le mica, la silice, le kaolin, les poudres de Nylon (Orgasol notamment) et de polyéthylène, le Téflon, l'amidon, le nitrure de bore, des microsphères de copolymères telles que l'Expancel (Nobel Industrie), le polytrap (Dow Corning) et les microbilles de résine de silicone (Tospearls de Toshiba, par exemple).

La composition selon l'invention peut être fabriquée classiquement par chauffage d'un mélange ou d'une pâte d'une ou plusieurs cires, d'une ou plusieurs silicones volatiles et éventuellement un ou plusieurs pigments, une ou plusieurs charges et/ou un ou plusieurs autres additifs à une température supérieure à la température la plus élevée de fusion des cires, puis coulage du mélange fondu dans un moule. Ce procédé permet d'obtenir une composition sous forme solide de stick ou de coupelle.

Elle peut aussi être obtenue par extrusion comme décrit dans la demande EP-A-667 146. Ce procédé consiste à malaxer la pâte (cires + volatile + additifs) pendant le refroidissement pour créer dans la masse des zones d'écrasement de la pâte à l'aide d'un broyeur à cylindres ou d'un extrudeur-mélangeur à vis. Ce procédé permet l'obtention d'une composition sous forme de pâte molle.

Par silicone volatile à chaîne alkyle, on entend une huile siliconée à chaîne alkyle susceptible de s'évaporer au contact de la peau ou des lèvres. Ces silicones volatiles présentent les avantages de ne pas avoir un point éclair trop bas (supérieur à 40°C) limitant les risques d'inflammation par rapport à certaines silicones volatiles cycliques, d'être douce à l'application, sans odeurs et surtout sans goût, ce qui est très important pour une application sur lèvres.

Les silicones volatiles de la composition selon l'invention sont les alkyl heptaméthyltrisiloxanes avec un groupe alkyle en $C_4$, $C_5$, $C_6$, $C_7$ et $C_8$ comme par exemple l'hexyl heptaméthyltrisiloxane de formule : $(CH_3)_3$-Si-O-Si$(CH_3)(C_6H_{13})$-O-Si$(CH_3)_3$ ; l'octyl heptaméthyltrisiloxane de formule : $(CH_3)_3$-Si-O-Si$(CH_3)(C_8H_{15})$-O-Si$(CH_3)_3$ ; et leurs mélanges. Elles peuvent être associées à d'autres silicones volatiles de formule (I) suivante :

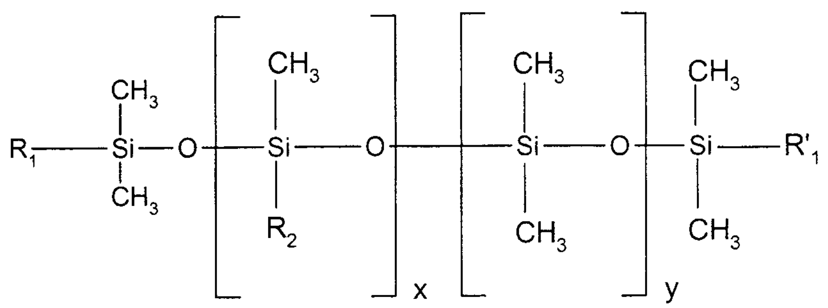
(I)

dans laquelle $R_1$, $R'_1$ et $R_2$ représentent indépendamment l'un de l'autre un groupe méthyle ou hydrogène ou une chaîne linéaire ou ramifiée ayant de 3 à 10 atomes de carbone, x et y représentent indépendamment un nombre entier

allant de 0 à 10 à condition que x soit différent de 0 quand $R_1$ et $R'_1$ représentent le groupe méthyle ou hydrogène avec $R_2$ différent de méthyle ou hydrogène et que $R_1$ ou $R'_1$ soit différent du groupe méthyle ou hydrogène quand $R_2$ représente un groupe méthyle ou hydrogène ou quand x vaut 0. En particulier, $R_2$ représente une chaîne propyle, butyle, pentyle, héxyle heptyle, ou octyle quand $R_1$ représente une chaîne méthyle et x représente 1, 2, 3, ou 4, de préférence 1 et y représente 0, 1, 2, 3, 4 et de préférence 0.

Avantageusement, la composition selon l'invention peut comprendre de 10 à 90 % du poids total de la composition, de préférence 40 à 80 %, d'une ou plusieurs silicones volatiles par rapport au poids total de la composition.

Les cires de silicone doivent être solides ou semi-solides à température ambiante. Ces cires peuvent se présenter sous forme d'une pâte ou d'un solide rigide. En particulier, ces cires présentes une température de fusion supérieure à 25 °C et mieux supérieure à 45 °C.

Les cires de silicone de la composition de l'invention peuvent présenter la formule (II) suivante :

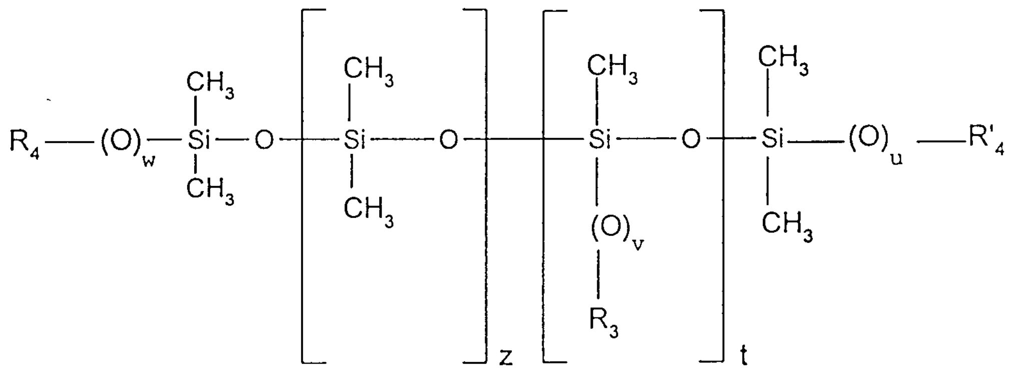
(II)

dans laquelle $R_3$, $R_4$ et $R'_4$ représentent indépendamment l'un de l'autre un groupe méthyle ou hydrogène ou une chaîne alkyle linéaire ou ramifiée ayant de 10 à 45 atomes de carbone, z et t représentent indépendamment un nombre entier allant de 0 à 100, u, v et w représentent indépendamment 0 ou 1, à condition que t soit différent de 0 et R3 différent de méthyle et hydrogène quand $R_4$ et $R'_4$ représentent le groupe méthyle ou hydrogène et que $R_4$ ou $R'_4$ soit différent du groupe méthyle ou hydrogène quand $R_3$ représente un groupe méthyle ou hydrogène ou quand t vaut 0. En particulier, $R_3$, $R_4$ ou $R'_4$ représente un chaîne linéaire ayant 12 à 35 atomes de carbone, et mieux de 18 à 28 atomes de carbone comme par exemple les radicaux $C_{16}H_{33}$, $C_{18}H_{37}$, $C_{24}H_{49}$, $C_{26}H_{53}$, ou un mélange de ces radicaux. De préférence $R_3$ est une chaine alkyleé et $R_4$ le groupe méthyle, u, v, et w sont égaux à 0, z vaut de 2 à 40 et t vaut de 45 à 98.

Parmi les cires de silicone utilisables dans l'invention, on peut citer la béhenoxy dimethicone (avec $R_4$=$CH_3(CH_2)_{21}$, t=0, u=1, w=1, z<10) telle que celle vendue par Goldschmidt sous le nom Abil Wax 2440 (température de fusion de 35°C) ; la stearyl dimethicone (avec u=0, v=w=0 $R_4$= CH3 et $R_3$= stéaryle) telle que celle vendue par Dow Corning sous le nom DC 2503 ; la cétyl dimethicone (avec u=v=w=0 , $R_4$= CH3 et $R_3$=cetyle) telle que celle vendue par Goldschmidt sous le nom Abil Wax 9814 ; la stearyl methicone (avec z=u=w=v=0 , $R_4$= CH3 et $R_3$=stearyle) telle que celle vendue par Goldschmidt sous le nom Abil Wax 9809 ; $C_{24}$-$C_{28}$ alkyl dimethicone (avec u=v=w=0 , $R_4$= CH3 et $R_3$ est un groupe alkyle en $C_{24}$-$C_{28}$ et z<5) telle que celle vendue par Goldschmidt sous le nom Abil Wax 9810 (température de fusion de 60°C) ; $C_{30}$-$C_{45}$ alkyl methicone (avec z=u=v=w=0 , $R_4$= CH3 et $R_3$=un groupe alkyle en $C_{30}$-$C_{45}$) telle que celle vendue par Goldschmidt sous le nom Abil Wax 9811 ; la stearoxy dimethicone (avec z=u=v=w=0 , $R_4$= CH3 et $R_3$=stearyle) telle que celle vendue par Goldschmidt sous le nom Abil Wax 2434 (température de fusion de 25°C et t=10) ;

On peut aussi utiliser les cires siliconées pour lesquelles $R_4$ et $R'_4$ sont le groupe méthyle, u=w=v=0 et les autres paramètres de la formule ainsi que la température de fusion de la cire sont donnés dans le tableau (I) ci-après :

| Cire référence | z | t | $R_4$ | température fusion |
|---|---|---|---|---|
| 1 | 60 | 40 | $C_{18}$ | 30 °C |
| 2 | 95 | 5 | > $C_{30}$ | 60 °C |

(suite)

| Cire référence | z | t | $R_4$ | température fusion |
|---|---|---|---|---|
| 3 | 90 | 10 | $C_{24}/C_{28}$ | 44 °C |
| 4 | 98 | 2 | $C_{24}/C_{28}$ | 41 °C |
| 5 | 95 | 5 | $C_{24}/C_{28}$ | 39 °C |
| 6 | 60 | 40 | $C_{24}/C_{28}$ | 57 °C |
| 7 | 60 | 40 | > $C_{30}$ | 60 °C |

Avantageusement, la composition selon l'invention peut comprendre de 2 à 90 % du poids total de la composition, de préférence 30 à 70 %, d'une ou plusieurs cires de silicones par rapport au poids total de la composition.
Comme autres cires de silicone utilisables dans l'invention, on peut citer les copolymères d'alkyl diméthicones.
Ces copolymères sont notamment ceux décrits dans les documents EP-A-527594, US-A-5 061 481, US-A-5397 566, EP-A-527594 et peuvent présenter la formule (III) suivante :

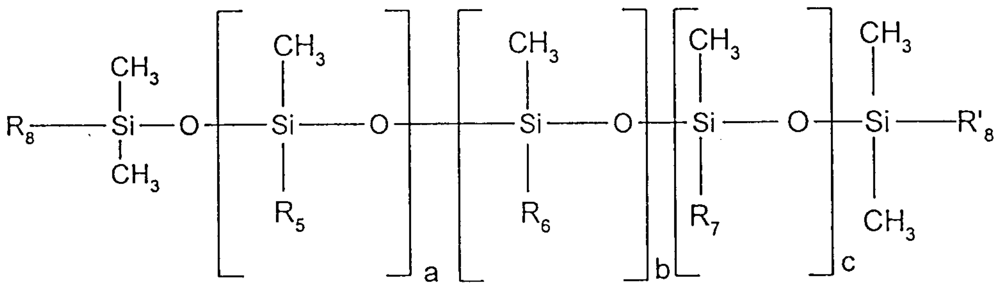 (III)

dans laquelle $R_5$ ,$R_6$ ,$R_7$, $R_8$ et $R'_8$ représentent indépendamment l'un de l'autre un groupe méthyle ou hydrogène ou une chaîne alkyle ou alcoxy linéaire ou ramifiée ayant de 5 à 36 atomes de carbone, a et b représentent indépendamment un nombre entier allant de 1 à 50 et c représente un nombre entier allant de 0 à 50, à condition que deux des radicaux $R_5$ ,$R_6$ ,$R_7$, $R_8$ et $R'_8$ soient différents du groupe méthyle ou hydrogène et soient différents l'un de l'autre. En particulier, $R_5$ ,$R_6$ représentent une chaîne linéaire ayant 10 à 20 atomes de carbone, avec $R_5$ différent de $R_6$, $R_8$ et $R'_8$ sont le groupe méthyle, a va de 8 à 18, b va 2 à 12, c vaut 0.
Comme copolymères utilisables dans l'invention, on peut citer les copolymères de formule (III) dans laquelle c vaut 0, $R_8$ et R'8 sont le groupe méthyle, les autres paramètres de la formule ainsi que la température de fusion de ces copolymères étant donnés dans le tableau (II) suivant :

| Copolymères | $R_5$ | % Ramification | x | $R_6$ | % Ramification | y | Température de fusion |
|---|---|---|---|---|---|---|---|
| $C_{01}$ | $C_{14}$ | 6 % | 10 | $C_{16}$ | 6 % | 10 | - |
| $C_{02}$ | $C_{14}$ | 6 % | 10 | $C_{18}$ | 6 % | 10 | 26 - 28 °C |
| $C_{03}$ | $C_{16}$ | 6 % | 10 | $C_{16}$-$C_{18}$ | 32 % | 10 | - |
| $C_{04}$ | $C_{18}$ | 6 % | 10 | $C_{16}$-$C_{18}$ | 32 % | 10 | - |
| $C_{05}$ | $C_{16}$ | 6 % | 18 | $C_{10}$ | 0 % | 2 | 25 - 27 °C |
| $C_{06}$ | $C_{16}$ | 6 % | 16 | $C_{10}$ | 0 % | 4 | - |

Ces copolymères peuvent représenter de 0 à 85 % du poids de la composition, et de préférence de 2 à 40 %
Ces copolymères de par leur structure asymétrique ($R_1$ différent de $R_2$) se présentent sous forme de cire élastique, de consistance intermédiaire entre cire et gomme (polydiméthylesiloxane de haut poids moléculaire) parfaitement miscibles aux alkyl diméthicones décrits précédemment. Ils renforcent l'apport de confort du film sans nuire à la propriété de ⟨⟨non transfert⟩⟩. Ces copolymères peuvent être utilisés seuls ou en mélange et avantageusement associés à une ou plusieurs cires de formule (II). Ces copolymères peuvent contribuer à la dureté du stick et/ou aux qualités cosmétiques.

La composition de l'invention peut, en outre, comprendre, en plus des silicones volatiles mentionnées ci-dessus, les corps gras usuellement utilisés dans le domaine d'application envisagé. Comme corps gras, on peut citer des silicones sous forme liquide estérifiées ou non ou sous forme solide estérifiées telles qu'une béhénate diméthicone, les corps gras non siliconés tels que les huiles, les pâteux et les cires végétales, minérales, animales et/ou synthétiques.

Parmi les corps gras non siliconés, on peut citer les huiles végétales comme l'huile de ricin, d'avocat, de jojoba ; des esters d'acides gras comme le myristate d'isopropyle ; des alcools comme l'actyl dodécanol ou l'alcool oléique ; des acétylglycérides ; des octanoates, décanoates ou ricinoléates d'alcools ou de polyalcools ; des triglycérides d'acides gras comme l'huile de sésame ; des lanolines ; des hydrocarbures comme la vaseline, l'huile de parléam, le polyisobutène ; la cire d'abeilles ; les cires végétales telles que la cire de Carnauba ou de Candellila; les cires minérales, par exemple de paraffine, de lignite ou les cires microcristallines ou les ozokérites ; les cires synthétiques comme les cires de polyéthylène.

Ces corps gras siliconés ou non peuvent être choisis de manière variée par l'homme du métier afin de préparer une composition ayant les propriétés, par exemple de consistance ou de texture, souhaitées.

En particulier, la composition selon l'invention peut comprendre au moins une des cires citées précédemment, de manière à assurer une bonne résistance mécanique, notamment lorsque la composition se présente sous la forme d'un stick.

D'une manière générale, la composition peut comprendre de 0 à 30% du poids total de la composition d'au moins une cire hydrocarbonée et de préférence de 5 à 25% en poids de cire hydrocarbonée.

De plus, on a constaté que l'amélioration de la tenue de la composition selon l'invention, ainsi que son absence de migration et/ou de transfert, pouvait être particulièrement intéressante lorsque la composition comprenait moins de 20% en poids d'huile non volatile hydrocarbonée, de préférence moins de 5% en poids, voire pas d'huile hydrocarbonée non volatile du tout.

La composition de l'invention peut comprendre, en outre, tout additif usuellement utilisé dans le domaine concerné, tel que des antioxydants, des parfums, des huiles essentielles, des conservateurs, des actifs cosmétiques, des hydratants, des vitamines, des acides gras essentiels, des filtres solaires lipophiles, des polymères liposolubles notamment hydrocarbonés tels que les polyalkylènes, les polyacrylates. Ces additifs peuvent être présents dans la composition à raison de 0 à 10% du poids total de la composition.

Bien entendu l'homme du métier veillera à choisir les éventuels composés complémentaires, et/ou leur quantité, de telle manière que les propriétés avantageuses de la composition selon l'invention ne soient pas, ou substantiellement pas, altérées par l'adjonction envisagée.

Les compositions selon l'invention peuvent se présenter notamment sous la forme de stick ou bâton, ou sous la forme de pâte souple ou coulée, voire sous la forme d'un liquide huileux gélifié ou d'une crème.

La composition selon l'invention peut se présenter sous la forme d'un produit de maquillage de la peau, en particulier un fond de teint, un fard à joues ou à paupières, ou mieux un rouge à lèvres. Elles peuvent également se présenter sous forme non colorée, contenant éventuellement des actifs cosmétiques ou dermatologiques. Elles peuvent alors être utilisées comme base de soin pour les lèvres ou base fixante à appliquer sur un rouge à lèvres classique. La base fixante forme alors un film protecteur sur le film de rouge, qui en limite le transfert et la migration.

La composition de l'invention peut également se présenter sous la forme d'une composition dermatologique ou de soin de la peau, ou sous forme d'une composition de protection solaire plus spécialement.

Bien entendu la composition de l'invention doit contenir un milieu cosmétiquement ou dermatologiquement acceptable, à savoir un milieu susceptible d'être appliqué sur la peau, les muqueuses (lèvres, intérieur des paupières) d'être humains.

L'invention a encore pour objet un rouge à lèvres ou un fond de teint anhydre sans transfert tel que défini dans les revendications.

L'invention a encore pour objet l'utilisation non-thérapeutique de l'association d'une silicone volatile à température ambiante choisie parmi l'hexyl heptaméthyltrisiloxane, l'octyl heptaméthyltrisiloxane et leurs mélanges, et d'une cire de silicone solide ou semi-solide à température ambiante, dans une composition cosmétique ou pour la fabrication d'une composition afin de diminuer le transfert et/ou la migration de ladite composition, la cire de silicone comportant une structure siliconée et des motifs à au moins une chaîne alkyle ou alcoxy pendante et/ou en bout de structure siliconée, ces chaînes étant linéaires ou ramifiées et ayant de 10 à 45 atomes de carbone.

L'invention a encore pour objet un procédé cosmétique pour limiter et/ou empêcher le transfert d'une composition de maquillage ou de soin de la peau ou des lèvres sur un substrat différent de la dite peau et desdites lèvres, le procédé étant défini dans les revendications.

L'invention est illustrée plus en détail dans les exemples suivants.

**Exemple 1 de rouge à lèvres** :

On prépare un bâton de rouge à lèvres ayant la composition suivante :

| | |
|---|---|
| . hexyl heptamethyltrisiloxane | qsp 100 g |
| . stearyl dimethicone ($T_f$=28°C) | 23 g |
| . cire de polyéthylène (Poly wax 500 de chez Bareco) | 23 g |
| . pigments | 8 g |
| . nacres naturelles | 2 g |

On prépare la composition de manière usuelle, en chauffant les corps gras, sauf la silicone volatile, à 110 °C et en les mélangeant. On ajoute ensuite les pigments et les charges, et, à 60 °C, les huiles volatiles. On mélange le tout avec une agitation douce. On peut alors couler ledit mélange dans des moules adéquats.

On obtient ainsi un bâton de rouge à lèvres, de texture agréable douce, qui s'étale bien et s'applique uniformément. Le film est confortable à porter dans le temps ; il ne migre pas ni ne transfère.

On applique cette composition sur la partie gauche des lèvres de plusieurs personnes.

Pour comparaison, on applique sur la partie droite desdites lèvres, un rouge à lèvres de l'art antérieur, ne comprenant pas de sire de silicone alkylée.

On laisse sécher les rouges à lèvres à température ambiante pendant 30 minutes, puis on applique la totalité des lèvres sur une feuille de papier.

On constate sur la totalité des feuilles de papier une trace de rouge à lèvres très faible, à peine perceptible, aussi bien pour la composition de l'invention que pour la composition de l'art antérieur. En outre, le film obtenu sur les lèvres avec la composition de l'invention est reconnu plus confortable et moins sec que celui obtenu avec la composition de l'art antérieur.

**Exemple 2 de rouge à lèvres** :

On prépare un bâton de rouge à lèvres ayant la composition suivante :

| | |
|---|---|
| . hexyl heptamethyltrisiloxane | qsp 100 g |
| . Abil Wax 9810 | 25 g |
| . silicone fluide à température ambiante (Abil Wax 9801) | 5 g |
| . pigments | 8 g |
| . mica-titane | 2 g |

On prépare la composition de manière usuelle, en chauffant les corps gras, à 95 °C, puis en ajoutant les pigments et les nacres sauf la silicone volatile, et en mélangeant le tout. On ajoute alors la silicone volatile à 60 °C puis on mélange le tout. On peut alors couler ce mélange dans des conditionnements adéquats.

On obtient ainsi un stick dure, de texture agréable, qui s'étale bien et s'applique uniformément. Le film obtenu sur les lèvres est confortable à porter dans le temps et ne migre pas.

**Exemple 3 de base fixante de rouge à lèvres** :

On prépare une base fixante pour rouge à lèvres ayant la composition suivante:

| | |
|---|---|
| . l'octyl heptaméthyltrisiloxane | qsp 100 g |
| . Abil Wax 9810 | 30 g |
| . cires de polyétylène (Poly wax 500 de chez Bareco) | 10 g |
| . charges (poudre de Nylon) | 5 g |

On prépare la composition selon l'exemple 1.
On obtient une base fixante sous forme de stick. Cette base est de texture agréable et s'applique aisément sur un film de rouge à lèvres classique. Elle permet d'éviter la migration et le transfert du film classique de rouge à lèvres sur un support tel qu'un verre.

**Exemple 4 de fond de teint coulé** :

On prépare un fond de teint ayant la composition suivante :

| | |
|---|---|
| . stéaryl diméthicone (cire siliconée de $T_f$=35 à 40°C) | 13,5 g |
| . hexyl heptaméthyltrisiloxane | qsp 100 g |
| . pigments | 16,2 g |
| . charges (Orgasol) | 16,7 g |
| . conservateurs | qs |

On fond la cire, puis on introduit les autres constituants dans l'ordre suivant : pigments, charges, conservateurs puis la silicone volatile et on mélange le tout. On peut alors couler ledit mélange dans des moules adéquats.
On obtient un fond de teint coulé 《 non transfert 》que l'on peut étaler à l'éponge, d'une grande douceur et d'étalement facile.

**Revendications**

1. Composition de maquillage ou de soin sans transfert, caractérisée en ce qu'elle contient au moins une silicone volatile à température ambiante choisie parmi l'hexyl heptaméthyltrisiloxane, l'octyl heptaméthyltrisiloxane et leurs mélanges, et une cire de silicone solide ou semi-solide à température ambiante comportant une structure siliconée et des motifs à au moins une chaîne alkyle ou alcoxy pendante et/ou en bout de structure siliconée, ces chaînes étant linéaires ou ramifiées et ayant de 10 à 45 atomes de carbone.

2. Composition selon la revendication 1, caractérisée en ce que la composition est sous forme anhydre.

3. Composition selon la revendication 1 ou 2, caractérisée en ce que la silicone volatile est présente à raison de 10 à 90% en poids, de préférence 40 à 80 %, du poids total de la composition.

4. Composition selon l'une des revendications précédentes, caractérisée en ce que la cire de silicone présente la formule (II) suivante :

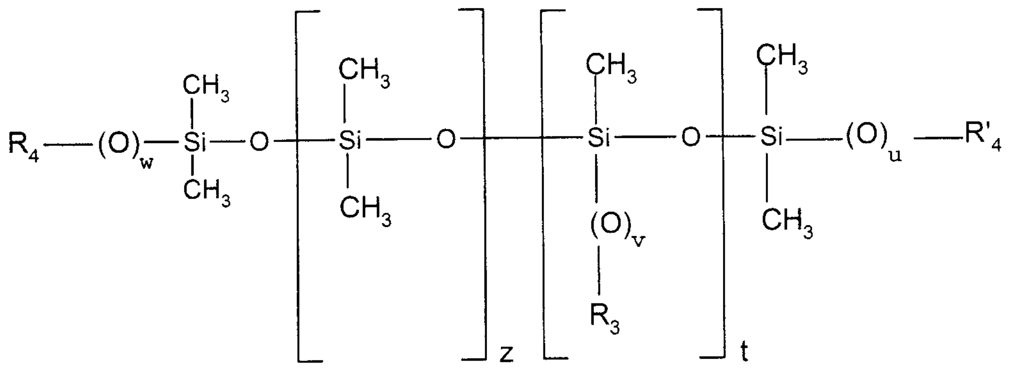 (II)

dans laquelle $R_3$, $R_4$ et $R'_4$ représentent indépendamment l'un de l'autre un groupe méthyle ou hydrogène ou une chaîne alkyle linéaire ou ramifiée ayant de 10 à 45 atomes de carbone, z et t représentent indépendamment un nombre entier allant de 0 à 100, u, v et w représentent indépendamment 0 ou 1, à condition que t soit différent de 0 et $R_3$ différent de méthyle ou hydrogène quand $R_4$ et $R'_4$ représentent le groupe méthyle ou hydrogène et que R4 ou R'4 soit différent du groupe méthyle ou hydrogène quand $R_3$ représente un groupe méthyle ou hydrogène ou quand t vaut 0.

5. Composition selon l'une des revendications précédentes, caractérisée en ce que la cire de silicone est choisie parmi les cires de formule (II) dans lesquelles $R_3$, $R_4$ ou $R'_4$ est un radical $C_{16}H_{33}$, $C_{18}H_{37}$, $C_{24}H_{49}$, $C_{26}H_{53}$, ou un mélange de ces radicaux.

6. Composition selon l'une des revendications précédentes, caractérisée en ce que la cire silicone est présente à raison de 2 à 90 % du poids total de la composition, de préférence 30 à 70 %.

7. Composition selon l'une des revendications précédentes, caractérisée en ce qu'elle contient moins de 5 % du poids total de la composition en huile hydrocarbonée non volatile.

8. Composition selon l'une des revendications précédentes, caractérisée en ce que la composition comprend, en outre, de 0 à 30 % du poids total de la composition de cire hydrocarbonée, de préférence 5 à 25 % en poids.

9. Composition selon l'une des revendications précédentes, caractérisée en ce qu'elle comprend, en outre, une phase particulaire présente à raison de 0 à 35 % du poids total de la composition, de préférence 5 à 25 %.

10. Composition selon l'une des revendications précédentes, caractérisée en ce qu'elle se présente sous la forme de stick ou bâton, de pâte souple ou coulée, ou d'une crème ou d'un gel.

11. Composition selon l'une des revendications précédentes, caractérisée en ce qu'elle contient, en outre, un actif cosmétique ou dermatologique.

12. Composition selon l'une des revendications précédentes, caractérisée en ce qu'elle se présente sous forme d'une composition de fond de teint, de fard à joues ou à paupières, ou d'un rouge à lèvres, d'une base de soin ou d'une base fixante pour les lèvres, d'un produit de soin de la peau ou d'une composition de protection solaire.

13. Composition selon l'une des revendications précédentes, caractérisée en ce qu'elle contient, en outre, un copolymère siliconé de formule (III) :

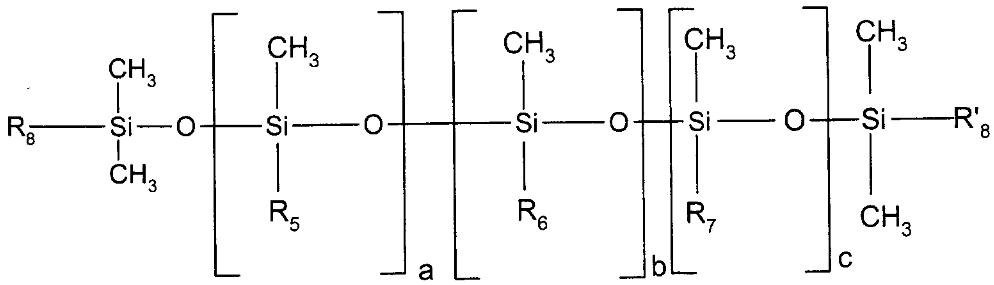

(III)

dans laquelle $R_5$ ,$R_6$ ,$R_7$, $R_8$ et $R'_8$ représentent indépendamment l'un de l'autre un groupe méthyle ou hydrogène ou une chaîne alkyle ou alcoxy linéaire ou ramifiée ayant de 5 à 36 atomes de carbone, a et b représentent indépendamment un nombre entier allant de 1 à 50 et c représente un nombre entier allant de 0 à 50, à condition que deux des radicaux $R_5$ ,$R_6$ ,$R_7$, $R_8$ et $R'_8$ soient différents du groupe méthyle ou hydrogène et soient différents l'un de l'autre.

**14.** Composition selon la revendication 13, caractérisée en ce que le copolymère représente de 0 à 85 % du poids de la composition, et de préférence de 2 à 40 %.

**15.** Rouge à lèvres ou fond de teint anhydre sans transfert, caractérisé en ce qu'il contient au moins une silicone volatile à température ambiante choisie parmi l'hexyl heptaméthyltrisiloxane, l'octyl heptaméthyltrisiloxane et leurs mélanges et une cire de silicone solide ou semi-solide à température ambiante comportant une structure siliconée et des motifs à au moins une chaîne alkyle ou alcoxy pendante et/ou en bout de structure siliconée, ces chaînes étant linéaires ou ramifiées et ayant de 10 à 45 atomes de carbone, et des pigments et/ou des charges.

**16.** Utilisation non-thérapeutique de l'association d'une silicone volatile à température ambiante choisie parmi l'hexyl heptaméthyltrisiloxane, l'octyl heptaméthyltrisiloxane et leurs mélanges et d'une cire de silicone solide ou semi-solide à température ambiante, dans une composition cosmétique afin de diminuer le transfert et/ou la migration de ladite composition, la cire de silicone comportant une structure siliconée et des motifs à au moins une chaîne alkyle ou alcoxy pendante et/ou en bout de structure siliconée, ces chaînes étant linéaires ou ramifiées et ayant de 10 à 45 atomes de carbone.

**17.** Utilisation de l'association d'une silicone volatile à température ambiante choisie parmi l'hexyl heptaméthyltrisiloxane, l'octyl heptaméthyltrisiloxane et leurs mélanges et d'une cire de silicone solide ou semi-solide à température ambiante, pour la fabrication d'une composition afin de diminuer le transfert et/ou la migration de ladite composition, la cire de silicone comportant une structure siliconée et des motifs à au moins une chaîne alkyle ou alcoxy pendante et/ou en bout de structure siliconée, ces chaînes étant linéaires ou ramifiées et ayant de 10 à 45 atomes de carbone.

**18.** Utilisation selon la revendication16 ou 17, caractérisée en ce que la silicone volatile est présente à raison de 10 à 90 % en poids, de préférence 40 à 80 %, du poids total de la composition.

**19.** Utilisation selon l'une des revendications 16 à 18, caractérisée en ce que la cire de silicone a la formule (II) :

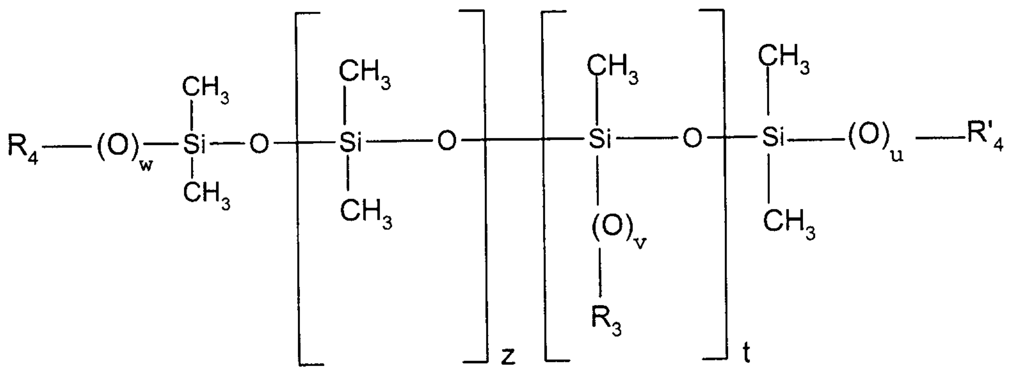 (II)

dans laquelle $R_3$, $R_4$ et $R'_4$ représentent indépendamment l'un de l'autre un groupe méthyle ou hydrogène ou une chaîne alkyle linéaire ou ramifiée ayant de 10 à 45 atomes de carbone, z et t représentent indépendamment un nombre entier allant de 0 à 100, u, v et w représentent indépendamment 0 ou 1, à condition que t soit différent de 0 et $R_3$ différent de méthyle ou hydrogène quand $R_4$ et $R'_4$ représentent le groupe méthyle ou hydrogène et que R4 ou R'4 soit différent du groupe méthyle ou hydrogène quand $R_3$ représente un groupe méthyle ou hydrogène et quand t vaut 0.

**20.** Utilisation selon l'une des revendications 16 à 19, caractérisée en ce que la cire de silicone est choisie parmi les cires de formule (II) dans lesquelles $R_3$, $R_4$ ou $R'_4$ est un radical $C_{16}H_{33}$, $C_{18}H_{37}$, $C_{24}H_{49}$, $C_{26}H_{53}$, ou un mélange de ces radicaux.

**21.** Utilisation selon l'une des revendications 16 à 20, caractérisée en ce que la cire silicone est présente à raison de 2 à 90 % du poids total de la composition, de préférence 30 à 70 %.

**22.** Utilisation selon l'une des revendications 16 à 21 caractérisée en ce que la composition comprend, en outre, de 0 à 30 % du poids total de la composition de cire hydrocarbonée, de préférence 5 à 25 % en poids.

**23.** Utilisation selon l'une des revendications 16 à 22, caractérisée en ce qu'elle comprend, en outre, une phase particulaire présente à raison de 0 à 35 % du poids total de la composition, de préférence 5 à 25 %.

**24.** Utilisation selon l'une des revendications 16 à 23, caractérisée en ce qu'elle se présente sous la forme de stick ou bâton, de pâte souple ou coulée, ou d'une crème ou d'un gel.

**25.** Utilisation selon l'une des revendications 16 à 24, caractérisée en ce qu'elle contient, en outre, un actif cosmétique ou dermatologique.

**26.** Utilisation selon l'une des revendications 16 à 25, caractérisée en ce qu'elle se présente sous forme d'une composition de fond de teint, de fard à joues ou a paupières, ou de rouge à lèvres, dans une base de soin ou une base fixante pour les lèvres, d'un produit de soin de la peau ou d'une composition solaire.

**27.** Utilisation selon l'une des revendications 16 à 26, caractérisée en ce qu'elle contient, en outre, un copolymère siliconé de formule (III) :

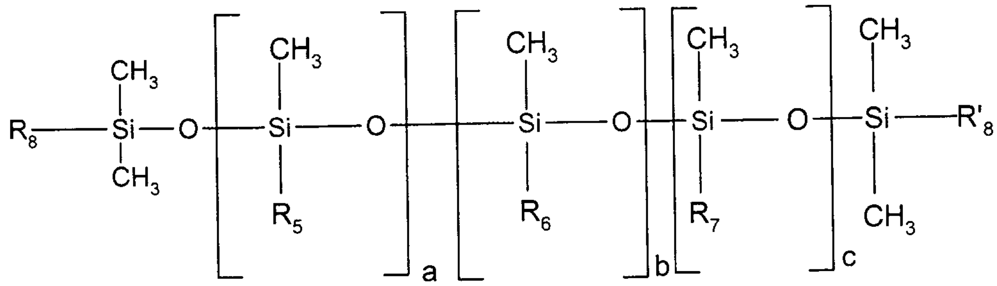

(III)

dans laquelle $R_5$ ,$R_6$ ,$R_7$, $R_8$ et $R'_8$ représentent indépendamment l'un de l'autre un groupe méthyle ou hydrogène ou une chaîne alkyle ou alcoxy linéaire ou ramifiée ayant de 5 à 36 atomes de carbone, a et b représentent indépendamment un nombre entier allant de 1 à 50 et c représente un nombre entier allant de 0 à 50, à condition que deux des radicaux $R_5$ ,$R_6$ ,$R_7$, $R_8$ et $R'_8$ soient différents du groupe méthyle ou hydrogène et soient différents l'un de l'autre.

**28.** Utilisation selon l'une des revendications 16 à 27, caractérisée en ce que le copolymère représente de 0 à 85 % du poids de la composition, et de préférence de 2 à 40 %.

**29.** Utilisation selon l'une des revendications 16 à 28, caractérisée en ce que la composition est anhydre.

**30.** Procédé cosmétique pour limiter et/ou empêcher le transfert d'une composition de maquillage ou de soin de la peau ou des lèvres sur un substrat différent de la dite peau et desdites lèvres, consistant à introduire dans la composition l'association d'une silicone volatile à température ambiante et d'une cire de silicone solide ou semi-solide à température ambiante, afin de diminuer le transfert et/ou la migration de ladite composition, la silicone volatile étant choisie parmi l'hexyl heptaméthyltrisiloxane, l'octyl heptaméthyltrisiloxane et leurs mélanges et la cire de silicone comportant une structure siliconée et des motifs à au moins une chaîne alkyle ou alcoxy pendante et/ou en bout de structure siliconée, ces chaînes étant linéaires ou ramifiées et ayant de 10 à 45 atomes de carbone

**Claims**

**1.** Transfer-resistant make-up or care composition characterized in that it contains at least one silicone which is volatile at ambient temperature, chosen from hexylheptamethyltrisiloxane, octylheptamethyltrisiloxane and mixtures thereof, and a silicone wax which is solid or semisolid at ambient temperature, comprising a silicone structure and units containing at least one alkyl or alkoxy chain which is pendent and/or at the end of the silicone structure, these chains being linear or branched and containing from 10 to 45 carbon atoms.

**2.** Composition according to Claim 1, characterized in that the composition is in anhydrous form.

**3.** Composition according to Claim 1 or 2, characterized in that the volatile silicone is present in a proportion of 10 to 90 % by weight, preferably 40 to 80 %, of the total weight of the composition.

**4.** Composition according to one of the preceding claims, characterized in that the silicone wax has the following formula (II):

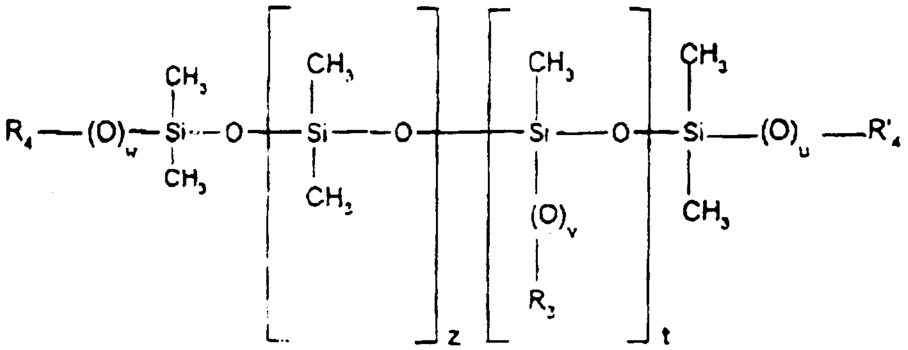

(II)

in which $R_3$, $R_4$ and $R'_4$ denote, independently of one another, a methyl group or hydrogen or a linear or branched alkyl chain containing from 10 to 45 carbon atoms, z and t denote independently an integer ranging from 0 to 100, u, v and w denote independently 0 or 1, on condition that t is other than 0 and $R_3$ other than methyl or hydrogen when $R_4$ and $R'_4$ denote the methyl group or hydrogen and that $R_4$ or $R'_4$ is other than the methyl group or hydrogen when $R_3$ denotes a methyl group or hydrogen or when t has the value 0.

5. Composition according to one of the preceding claims, characterized in that the silicone wax is chosen from the waxes of formula (II) in which $R_3$, $R_4$ or $R'_4$ is a radical $C_{16}H_{33}$, $C_{18}H_{37}$, $C_{24}H_{49}$ or $C_{26}H_{53}$ or a mixture of these radicals.

6. Composition according to one of the preceding claims, characterized in that the silicone wax is present in a proportion of 2 to 90 % of the total weight of the composition, preferably 30 to 70 %.

7. Composition according to one of the preceding claims, characterized in that it contains less than 5 % of the total weight of the composition of nonvolatile hydrocarbon oil.

8. Composition according to one of the preceding claims, characterized in that the composition additionally includes from 0 to 30 % of the total weight of the composition of hydrocarbon wax, preferably 5 to 25 % by weight.

9. Composition according to one of the preceding claims, characterized in that it additionally includes a particulate phase present in a proportion of 0 to 35 % of the total weight of the composition, preferably 5 to 25 %.

10. Composition according to one of the preceding claims, characterized in that it is presented in the form of a stick or cake, of flexible or cast paste or of a cream or a gel.

11. Composition according to one of the preceding claims, characterized in that it additionally contains a cosmetic or dermatological active substance.

12. Composition according to one of the preceding claims, characterized in that it is presented in the form of a foundation, blusher or eyeshadow composition or of a lip rouge, of a care base or a fixing base for the lips, of a skin care product or of a composition for solar protection.

13. Composition according to one of the preceding claims, characterized in that it additionally contains a silicone copolymer of formula (III):

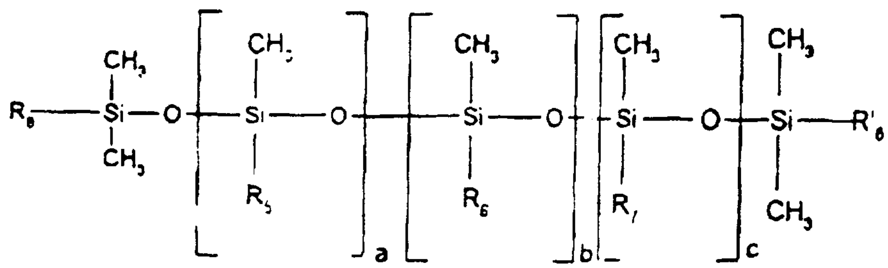

(III)

in which $R_5$, $R_6$, $R_7$, $R_8$ and $R'_8$ denote, independently of one another, a methyl group or hydrogen or a linear or branched alkyl or alkoxy chain containing from 5 to 36 carbon atoms, a and b independently denote an integer ranging from 1 to 50 and c denotes an integer ranging from 0 to 50, on condition that two of the radicals $R_5$, $R_6$, $R_7$, $R_8$ and $R'_8$ are other than the methyl group or hydrogen and differ from one another.

**14.** Composition according to Claim 13, characterized in that the copolymer represents from 0 to 85 % of the weight of the composition, and preferably from 2 to 40 %.

**15.** Transfer-resistant anhydrous lip rouge or foundation characterized in that it contains at least one silicone which is volatile at ambient temperature, chosen from hexylheptamethyltrisiloxane, octylheptamethyltrisiloxane and mixtures thereof, and a silicone wax which is solid or semisolid at ambient temperature, comprising a silicone structure and units containing at least one alkyl or alkoxy chain which is pendent and/or at the end of the silicone structure, these chains being linear or branched and containing from 10 to 45 carbon atoms, and pigments and/or fillers.

**16.** Non-therapeutic use of a combination of a silicone which is volatile at ambient temperature, chosen from hexylheptamethyltrisiloxane, octylheptamethyltrisiloxane and mixtures thereof, and of a silicone wax which is solid or semisolid at ambient temperature, in a cosmetic composition in order to decrease the transfer and/or the migration of the said composition, the silicone wax comprising a silicone structure and units containing at least one alkyl or alkoxy chain which is pendent and/or at the end of the silicone structure, these chains being linear or branched and containing from 10 to 45 carbon atoms.

**17.** Use of a combination of a silicone which is volatile at ambient temperature, chosen from hexylheptamethyltrisiloxane, octylheptamethyltrisiloxane and mixtures thereof, and of a silicone wax which is solid or semisolid at ambient temperature, for the manufacture of a composition in order to decrease the transfer and/or the migration of the said composition, the silicone wax comprising a silicone structure and units containing at least one alkyl or alkoxy chain which is pendent and/or at the end of the silicone structure, these chains being linear or branched and containing from 10 to 45 carbon atoms.

**18.** Use according to Claim 16 or 17, characterized in that the volatile silicone is present in a proportion of 10 to 90 % by weight, preferably 40 to 80 %, of the total weight of the composition.

**19.** Use according to one of Claims 16 to 18, characterized in that the silicone wax has the formula (II):

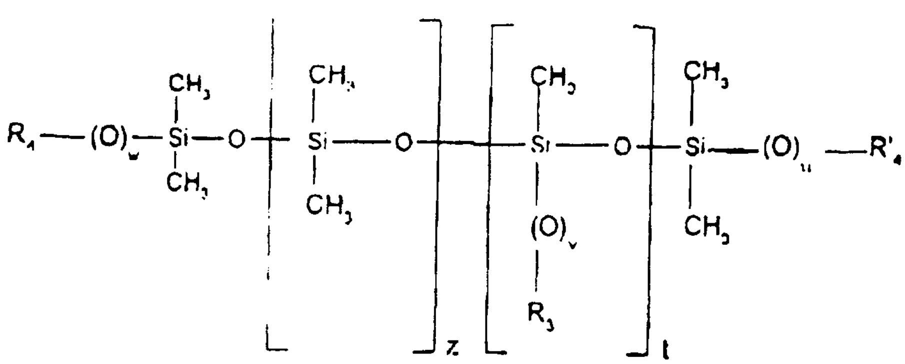

(II)

in which $R_3$, $R_4$ and $R'_4$ denote, independently of one another, a methyl group or hydrogen or a linear or branched alkyl chain containing from 10 to 45 carbon atoms, z and t denote independently an integer ranging from 0 to 100, u, v and w denote independently 0 or 1, on condition that t is other than 0 and $R_3$ other than methyl or hydrogen when $R_4$ and $R'_4$ denote the methyl group or hydrogen and that $R_4$ or $R'_4$ is other than the methyl group or hydrogen when $R_3$ denotes a methyl group or hydrogen or when t has the value 0.

**20.** Use according to one of Claims 16 to 19, characterized in that the silicone wax is chosen from the waxes of formula (II) in which $R_3$, $R_4$ or $R'_4$ is a radical $C_{16}H_{33}$, $C_{18}H_{37}$, $C_{24}H_{49}$ or $C_{26}H_{53}$ or a mixture of these radicals.

**21.** Use according to one of Claims 16 to 20, characterized in that the silicone wax is present in a proportion of 2 to 90 % of the total weight of the composition, preferably 30 to 70 %.

**22.** Use according to one of Claims 16 to 21, characterized in that the composition additionally includes from 0 to 30 % of the total weight of the composition of hydrocarbon wax, preferably 5 to 25 % by weight.

**23.** Use according to one of Claims 16 to 22, characterized in that it additionally includes a particulate phase present in a proportion of 0 to 35 % of the total weight of the composition, preferably 5 to 25 %.

**24.** Use according to one of Claims 16 to 23, characterized in that it is presented in the form of a stick or cake, of flexible or cast paste or of a cream or a gel.

**25.** Use according to one of Claims 16 to 24, characterized in that it additionally contains a cosmetic or dermatological active substance.

**26.** Use according to one of Claims 16 to 25, characterized in that it is presented in the form of a foundation, blusher or eyeshadow or lip rouge composition, in a care base or a fixing base for the lips, of a skin care product or of a solar composition.

**27.** Use according to one of Claims 16 to 26, characterized in that it additionally contains a silicone copolymer of formula (III):

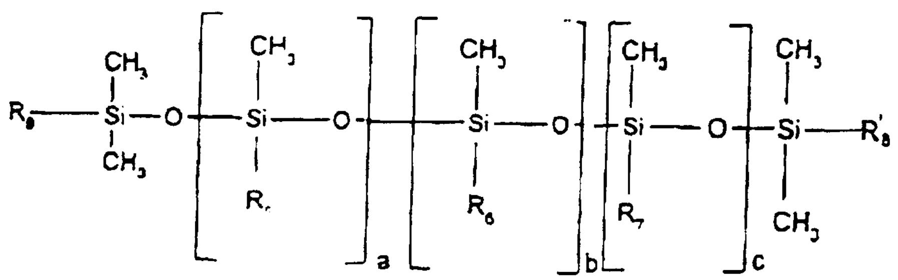

(III)

in which $R_5$, $R_6$, $R_7$, $R_8$ and $R'_8$ denote, independently of one another, a methyl group or hydrogen or a linear or branched alkyl or alkoxy chain containing from 5 to 36 carbon atoms, a and b independently denote an integer ranging from 1 to 50 and c denotes an integer ranging from 0 to 50, on condition that two of the radicals $R_5$, $R_6$, $R_7$, $R_8$ and $R'_8$ are other than the methyl group or hydrogen and differ from one another.

**28.** Use according to one of Claims 16 to 27, characterized in that the copolymer represents from 0 to 85 % of the weight of the composition, and preferably from 2 to 40 %.

**29.** Use according to one of Claims 16 to 28, characterized in that the composition is anhydrous.

**30.** Cosmetic process for limiting and/or preventing the transfer of a skin or lip make-up or care composition onto a substrate other than the said skin and the said lips, consisting in introducing into the composition the combination of a silicone which is volatile at ambient temperature and of a silicone wax which is solid or semisolid at ambient temperature, in order to decrease the transfer and/or the migration of the said composition, the volatile silicone being chosen from hexylheptamethyltrisiloxane, octylheptamethyltrisiloxane and mixtures thereof, and the silicone wax comprising a silicone structure and units containing at least one alkyl or alkoxy chain which is pendent and/or at the

end of the silicone structure, these chains being linear or branched and containing from 10 to 45 carbon atoms.

**Patentansprüche**

1. Zusammensetzung zum Schminken oder zur Pflege ohne Materialübertragung, dadurch gekennzeichnet, daß sie mindestens ein bei Umgebungstemperatur flüchtiges Silicon, das unter Hexylheptamethyltrisiloxan, Octylheptamethyltrisiloxan und ihren Gemischen ausgewählt ist, und ein bei Umgebungstemperatur festes oder halbfestes Siliconwachs enthält, das eine Siliconstruktur und Einheiten mit mindestens einer Alkyl- oder Alkoxykette aufweist, die als Seitenkette und/oder am Ende der Siliconstruktur angebunden ist, wobei diese Ketten geradkettig oder verzweigt sind und 10 bis 45 Kohlenstoffatome aufweisen.

2. Zusammensetzung nach Anspruch 1, dadurch gekennzeichnet, daß die Zusammensetzung in wasserfreier Form vorliegt.

3. Zusammensetzung nach Anspurch 1 oder 2, dadurch gekennzeichnet, daß das flüchtige Silicon in einem Anteil von 10 bis 90 Gew.-%, vorzugsweise 40 bis 80 Gew.-%, des Gesamtgewichts der Zusammensetzung enthalten ist.

4. Zusammensetzung nach einem der vorhergehenden Ansprüche, dadurch gekennzeichnet, daß das Siliconwachs die folgende Formel (II)

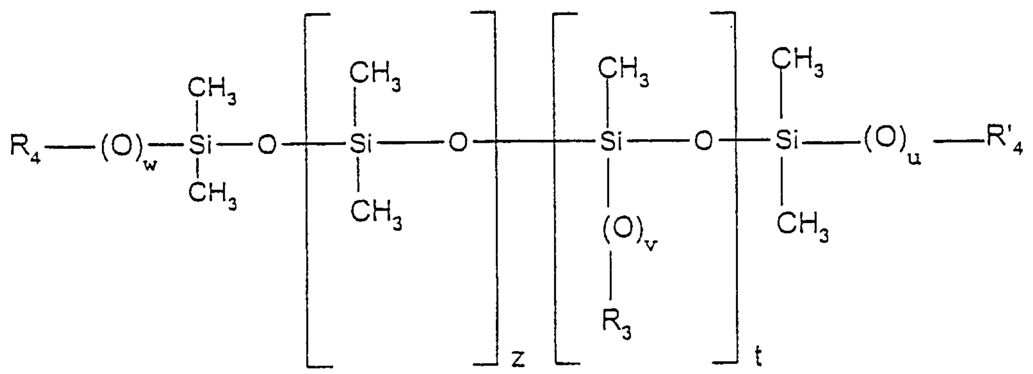 (II)

aufweist, in der bedeuten: $R_3$, $R_4$ und $R'_4$ unabhängig Methyl oder Wasserstoff oder geradkettiges oder verzweigtes Alkyl mit 10 bis 45 Kohlenstoffatomen, z und t unabhängig eine ganze Zahl von 0 bis 100, u, v und w unabhängig 0 oder 1, mit der Maßgabe, daß t verschieden von 0 und $R_3$ verschieden von Methyl und Wasserstoff ist, wenn $R_4$ und $R'_4$ Methyl oder Wasserstoff darstellen, und daß $R_4$ und $R'_4$ verschieden von Methyl oder Wasserstoff sind, wenn $R_3$ Methyl oder Wasserstoff darstellt oder wenn t gleich 0 ist.

5. Zusammensetzung nach einem der vorhergehenden Ansprüche, dadurch gekennzeichnet, daß das Siliconwachs unter den Wachsen der Formel (II) ausgewählt ist, in denen $R_3$, $R_4$ oder $R'_4$ den Rest $C_{16}H_{33}$, $C_{18}H_{37}$, $C_{24}H_{49}$, $C_{26}H_{53}$ oder ein Gemisch dieser Reste bedeutet.

6. Zusammensetzung nach einem der vorhergehenden Ansprüche, dadurch gekennzeichnet, daß das Siliconwachs in einem Anteil von 2 bis 90 % des Gesamtgewichts der Zusammensetzung und vorzugsweise in einem Anteil von 30 bis 70 % enthalten ist.

7. Zusammensetzung nach einem der vorhergehenden Ansprüche, dadurch gekennzeichnet, daß sie weniger als 5 Gew.-%, bezogen auf das Gesamtgewicht der Zusammensetzung, nicht flüchtiges Kohlenwasserstofföl enthält.

8. Zusammensetzung nach einem der vorhergehenden Ansprüche, dadurch gekennzeichnet, daß die Zusammensetzung außerdem Kohlenwasserstoffwachs in einem Anteil von 0 bis 30 % des Gesamtgewichts der Zusammensetzung, vorzugsweise in einem Anteil von 5 bis 25 %, enthält.

9. Zusammensetzung nach einem der vorhergehenden Ansprüche, dadurch gekennzeichnet, daß sie außerdem eine Partikel-Phase enthält, die in einem Anteil von 0 bis 35 % des Gesamtgewichts der Zusammensetzung, vorzugsweise in einem Anteil von 5 bis 25 %, enthalten ist.

**10.** Zusammensetzung nach einem der vorhergehenden Ansprüche, dadurch gekennzeichnet, daß sie in Form eines Sticks oder Stifts, einer weichen oder gegossenen Paste oder einer Creme oder eines Gels vorliegt.

**11.** Zusammensetzung nach einem der vorhergehenden Ansprüche, dadurch gekennzeichnet, daß sie außerdem einen kosmetischen oder dermatologischen Wirkstoff enthält.

**12.** Zusammensetzung nach einem der vorhergehenden Ansprüche, dadurch gekennzeichnet, daß sie in Form einer Make-up-, Rouge- oder Lidschatten-Zusammensetzung, eines Lippenstifts, einer Pflegegrundlage oder einer fixierenden Grundlage für die Lippen, eines Hautpflegemittels oder einer Sonnenschutzzusammensetzung vorliegt.

**13.** Zusammensetzung nach einem der vorhergehenden Ansprüche, dadurch gekennzeichnet, daß sie außerdem ein Silicon-Copolymer der folgenden Formel (III)

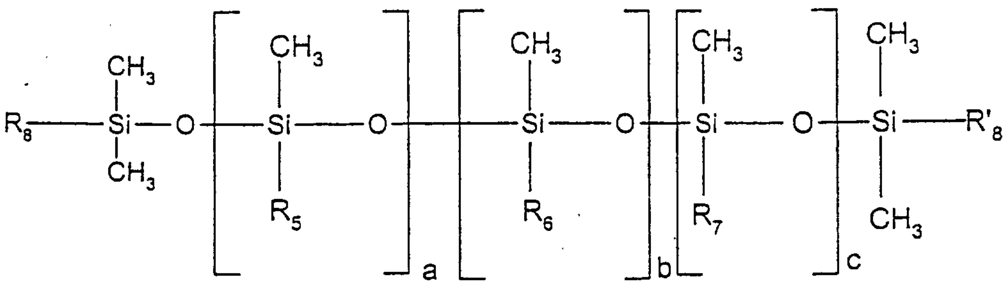

(III)

enthält, in der bedeuten: $R_5$, $R_6$, $R_7$, $R_8$ und $R'_8$ unabhängig Methyl oder Wasserstoff oder eine geradketttige oder verzweigte Alkyl- oder Alkoxykette mit 5 bis 36 Kohlenstoffatomen, a und b unabhängig eine ganze Zahl von 1 bis 50 und c eine ganze Zahl von 0 bis 50 mit der Maßgabe, daß zwei der Reste $R_5$, $R_6$, $R_7$, $R_8$ und $R'_8$ verschieden von Methyl oder Wasserstoff sind und verschieden voneinander sind.

**14.** Zusammensetzung nach Anspruch 13, dadurch gekennzeichnet, daß das Copolymer 0 bis 85 % des Gewichts der Zusammensetzung und vorzugsweise 2 bis 40 % ausmacht.

**15.** Wasserfreier Lippenstift oder wasserfreies Make-up ohne Materialübertragung, dadurch gekennzeichnet, daß sie mindestens ein bei Umgebungstemperatur flüchtiges Silicon, das unter Hexylheptamethyltrisiloxan, Octylheptamethyltrisiloxan und ihren Gemischen ausgewählt ist, ein bei Umgebungstemperatur festes oder halbfestes Siliconwachs, das eine Siliconstruktur und Einheiten mit mindestens einer Alkyl- oder Alkoxykette aufweist, die als Seitenkette und/oder am Ende der Siliconstruktur angebunden ist, wobei diese Ketten geradkettig oder verzweigt sind und 10 bis 45 Kohlenstoffatome aufweisen, und Pigmente und/oder Füllstoffe enthält.

**16.** Nicht-therapeutische Verwendung der Kombination eines bei Umgebungstemperatur flüchtigen Silicons, das unter Hexylheptamethyltrisiloxan, Octylheptamethyltrisoloxan und ihren Gemischen ausgewählt ist, und eines bei Umgebungstemperatur festen oder halbfesten Siliconwachses in einer kosmetischen Zusammensetzung oder für die Herstellung einer Zusammensetzung zur Verringerung der Materialübertragung und/oder der Migration der Zusammensetzung, wobei das Siliconwachs eine Siliconstruktur und Einheiten mit mindestens einer Alkyl oder Alkoxykette aufweist, die als Seitenkette und/oder am Ende der Siliconstruktur angebunden ist, wobei diese Ketten geradkettig oder verzweigt sind und 10 bis 45 Kohlenstoffatome aufweisen.

**17.** Verwendung der Kombination eines bei Umgebungstemperatur flüchtigen Silicons, das unter Hexylheptamethyltrisiloxan, Octylheptamethyltrisiloxan und ihren Gemischen ausgewählt ist, und eines bei Umgebungstemperatur festen oder halbfesten Siliconwachses zur Herstellung einer Zusammensetzung zur Verringerung der Materialübertragung und/oder der Migration der Zusammensetzung, wobei das Siliconwachs eine Siliconstruktur und Einheiten mit mindestens einer Alkyl oder Alkoxykette aufweist, die als Seitenkette und/oder am Ende der Siliconstruktur angebunden ist, wobei diese Ketten geradkettig oder verzweigt sind und 10 bis 45 Kohlenstoffatome aufweisen.

**18.** Verwendung nach Anspruch 16 oder 17, dadurch gekennzeichnet, daß das flüchtige Silicon in einem Anteil von 10 bis 90 Gew.-%, vorzugsweise 40 bis 80 %, des Gesamtgewichts der Zusammensetzung enthalten ist.

**19.** Verwendung nach einem der Ansprüche 16 bis 18, dadurch gekennzeichnet, daß das Siliconwachs die folgende Formel (II)

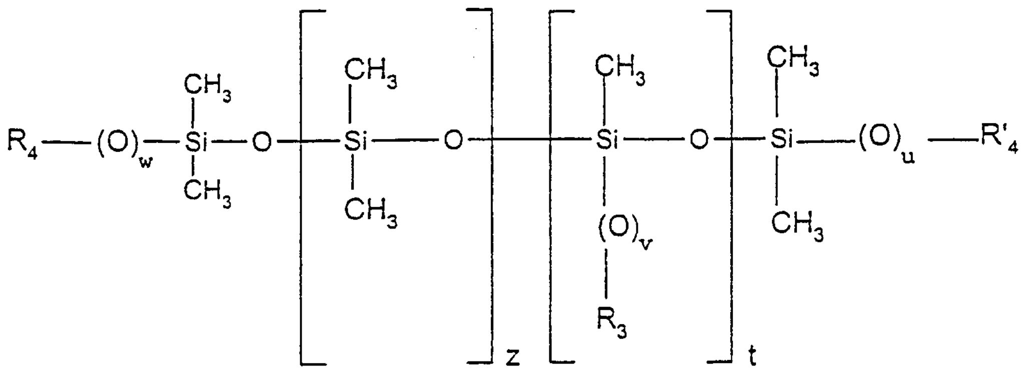

(II)

aufweist, in der bedeuten: $R_3$, $R_4$ und $R'_4$ unabhängig Methyl oder Wasserstoff oder geradkettiges oder verzweigtes Alkyl mit 10 bis 45 Kohlenstoffatomen, z und t unabhängig eine ganze Zahl von 0 bis 100, u, v und w unabhängig 0 oder 1, mit der Maßgabe, daß t verschieden von 0 und $R_3$ verschieden von Methyl und Wasserstoff ist, wenn $R_4$ und $R'_4$ Methyl oder Wasserstoff darstellen, und daß $R_4$ und $R'_4$ verschieden von Methyl oder Wasserstoff sind, wenn $R_3$ Methyl oder Wasserstoff darstellt oder wenn t gleich 0 ist.

**20.** Verwendung nach einem der Ansprüche 16 bis 19, dadurch gekennzeichnet, daß das Siliconwachs unter den Wachsen der Formel (II) ausgewählt ist, in denen $R_3$, $R_4$ oder $R'_4$ den Rest $C_{16}H_{33}$, $C_{18}H_{37}$, $C_{24}H_{49}$, $C_{26}H_{53}$ oder ein Gemisch dieser Reste bedeutet.

**21.** Verwendung nach einem der Ansprüche 16 bis 20, dadurch gekennzeichnet, daß das Siliconwachs in einem Anteil von 2 bis 90 % des Gesamtgewichts der Zusammensetzung und vorzugsweise in einem Anteil von 30 bis 70 % enthalten ist.

**22.** Verwendung nach einem der Ansprüche 16 bis 21, dadurch gekennzeichnet daß die Zusammensetzung außerdem Kohlenwasserstoffwachs in einem Anteil von 0 bis 30 % des Gesamtgewichts der Zusammensetzung, vorzugsweise in einem Anteil von 5 bis 25 %, enthält.

**23.** Verwendung nach einem der Ansprüche 16 bis 22 dadurch gekennzeichnet, daß die Zusammensetzung außerdem eine Partikel-Phase enthält, die in einem Anteil von 0 bis 35 % des Gesamtgewichts der Zusammensetzung, vorzugsweise in einem Anteil von 5 bis 25 %, enthalten ist.

**24.** Verwendung nach einem der Ansprüche 16 bis 23, dadurch gekennzeichnet, daß die Zusammensetzung in Form eines Sticks oder Stifts, einer weichen oder gegossenen Paste oder einer Creme oder eines Gels vorliegt.

**25.** Verwendung nach einem der Ansprüche 16 bis 24, dadurch gekennzeichnet, daß die Zusammensetzung außerdem einen kosmetischen oder dermatologischen Wirkstoff enthält.

**26.** Verwendung nach einem der Ansprüche 16 bis 25, dadurch gekennzeichnet, daß die Zusammensetzung in Form einer Make-up-, Rouge- oder Lidschatten-Zusammensetzung, eines Lippenstifts, einer Pflegegrundlage oder einer fixierenden Grundlage für die Lippen, eines Hautpflegemittels oder einer Sonnenschutzzusammensetzung vorliegt.

**27.** Verwendung nach einem der Ansprüche 16 bis 26, dadurch gekennzeichnet, daß die Zusammensetzung außerdem ein Silicon-Copolymer der folgenden Formel (III)

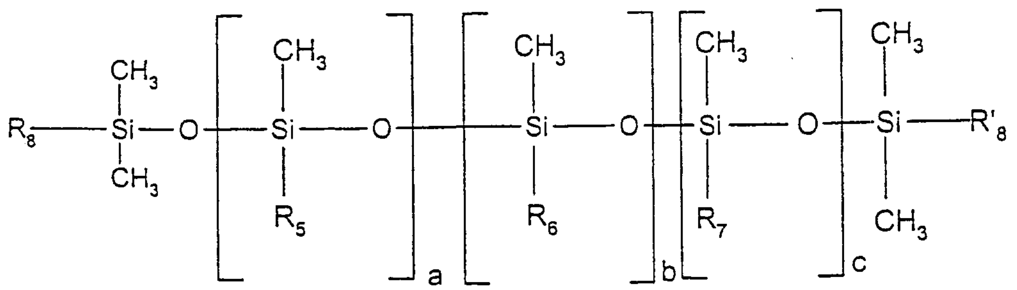

enthält, in der bedeuten: $R_5$, $R_6$, $R_7$, $R_8$ und $R'_8$ unabhängig Methyl oder Wasserstoff oder eine geradketttige oder verzweigte Alkyl- oder Alkoxykette mit 5 bis 36 Kohlenstoffatomen, a und b unabhängig eine ganze Zahl von 1 bis 50 und c eine ganze Zahl von 0 bis 50 mit der Maßgabe, daß zwei der Reste $R_5$, $R_6$, $R_7$, $R_8$ und $R'_8$ verschieden von Methyl oder Wasserstoff sind und verschieden voneinander sind.

**28.** Verwendung nach einem der Ansprüche 16 bis 27, dadurch gekennzeichnet, daß das Copolymer 0 bis 85 % des Gewichts der Zusammensetzung und vorzugsweise 2 bis 40 % ausmacht.

**29.** Verwendung nach einem der Ansprüche 16 bis 28, dadurch gekennzeichnet, daß die Zusammensetzung wasserfrei ist.

**30.** Kosmetisches Verfahren zur Begrenzung und/oder Verhinderung der Übertragung einer Zusammensetzung zum Schminken oder zur Pflege der Haut oder der Lippen auf einen von der Haut und den Lippen verschiedenen Träger, das darin besteht, in die Zusammensetzung die Kombination aus einem bei Umgebungstemperatur flüchtigen Silicon und einem bei Umgebungstemperatur festen oder halbfesten Siliconwachs einzubringen, um die Übertragung und/oder die Migration der Zusammensetzung zu verringern, wobei das flüchtige Silicon unter Hexylheptamethyltrisiloxan, Octylheptamethyltrisiloxan und ihren Gemischen ausgewählt ist und das Siliconwachs eine Siliconstruktur und Einheiten mit mindestens einer Alkyl oder Alkoxykette aufweist, die als Seitenkette und/oder am Ende der Siliconstruktur angebunden ist, wobei diese Ketten geradkettig oder verzweigt sind und 10 bis 45 Kohlenstoffatome aufweisen.